# NEUE EUROPÄISCHE PATENTSCHRIFT

(11) **EP 0 919 219 B2**
(45) Veröffentlichungstag und Bekanntmachung des Hinweises auf die Entscheidung über den Einspruch: **29.05.2013**
(45) Hinweis auf die Patenterteilung: 11.08.2004
(21) Anmeldenummer: 98120655.0
(22) Anmeldetag: 04.11.1998
(51) Int. Cl.: A61K 8/30, A61K 8/31, A61K 8/86, A61Q 5/06, A61Q 5/12

(54) **Verwendung eines Mittels zur Erhöhung der Formbarkeit und des Glanzes von Haaren**
Use of a composition for increasing formability and gloss of hairs
Utilisation d'un agent pour améliorer la mise en forme et la brillance des cheveux

(30) Priorität: 29.11.1997 DE 19753108
(43) Veröffentlichungstag der Anmeldung: 02.06.1999
(73) Patentinhaber: Wella GmbH, 65825 Schwalbach am Taunus (DE)
(72) Erfinder: Karlen, Thomas, Dr., 3013 Bern (CH); Chambettaz, Daniel, 1717 St. Ursen (CH); Steinbrecht, Karin, Dr., 64372 Ober-Ramstadt (DE); Irrgang, Bernhard, Dr., 1713 St. Anton (CH); Franzke, Michael, Dr., 64380 Rossdorf (DE)
(74) Vertreter: Holmes, Rosalind

(56) Entgegenhaltungen:
- EP-A1- 0 792 632
- WO-A-94/07458
- US-A- 5 597 551
- US-A- 5 626 835
- US-A- 6 056 947

## Beschreibung

Gegenstand der Erfindung ist die Verwendung eines bei Entnahme Schaumbildenden Mittels zur Erhöhung der Formbarkeit und des Glanzes von Haaren, das einen Fettsäureglyceridpolyalkylenglykolether oder einen Fettsäurepartialglyceridpolyalkylenglykolether mit 70 bis 250 Alkylenglykoleinheiten und ein Treibmittel, ausgewählt aus Dimethylether und gasförmigen Kohlenwasserstoffen oder deren Gemische, enthält.

Schon seit langem beschäftigt sich die Forschung auf dem Gebiet der Haarbehandlungsmittel mit Zubereitungen in Form von Pomaden, welche zur erleichterten Formgebung und zur Erhöhung des Glanzes und des Haltes der Frisur dienen. Zur Erzielung der festigenden und glanzerzeugenden Eigenschaften werden normalerweise Kohlenwasserstoffe (z.B. Paraffine), Siliconöle oder Tenside mit einem niedrigen HLB-Wert verwendet. Das Problem bei dieser Art von Pomaden liegt in der Stabilisierung der Formulierung, die zum Auftrennen in eine wässrige und in eine lipophile Phase neigt sowie in der schlechten Abbaubarkeit von Siliconölen.

Es besteht die Möglichkeit, übliche gut wasserlösliche nichtionische Tenside als Glanz- und Festigkeitsgeber zu verwenden wie zum Beispiel Fettalkoholethoxylate und Fettsäureethoxylate. Um solchen nichtionischen Tensiden eine ausreichende Wasserlöslichkeit zu verleihen, müssen sie in einer hochethoxylierten Form vorliegen. Zur Erzielung eines ausreichend starken Pomadeneffekts muß jedoch eine große Menge an hochethoxyliertem nichtionischen Tensid verwendet werden, was kostenintensiv ist. Zudem neigen hochethoxylierte nichtionische Tenside dazu, im wasserfreien Zustand wachsartig oder fest zu werden. Dies führt dazu, daß nach dem Auftragen aufs Haar und einer Trockenphase ein fester, trockener Film ausgebildet wird, der zu unangenehm hartem Griff und ungenügender Geschmeidigkeit der Haare führt.

Die Darreichungsform von Pomaden beschränkt sich üblicherweise weitgehend auf nichttransparente Massen in Form von cremeartigen Emulsionen. Diese Darreichungsform führt oft zu einer schlechten Verteilbarkeit der Masse auf dem Haar, wodurch die Frisurenerstellung erschwert wird. Eine verbesserte Applikationsmöglichkeit bietet sich dadurch, daß die Pomade in Form eines Aerosol-Schaums auf das Haar aufgetragen wird, der beim Einarbeiten in das Haar beziehungsweise beim Erstellen der Frisur zusammenbricht.

Nachteilig dabei ist jedoch, daß dieses Ziel mit den bisher verwendeten Haarbehandlungsmitteln nur in Form von Massen erreicht werden konnte, die sich innerhalb der zwischen zwei Anwendungen üblichen Zeit von ein bis mehreren Tagen in einen wässrigen Teil und einen Treibgasanteil trennen. Dadurch wurde die Bereitstellung eines einphasigen Produkts mit den entsprechenden Convenience-Vorteilen für den Verbraucher erschwert. Eine starke Erhöhung der Emulgatoranteile führt zwar zum gewünschten einphasigen Produkt, jedoch auch zu einer erheblichen Kostenerhöhung und zu einer starken Belastung des Haars. Die Verwendung von hohen Anteilen an nichtwässrigen Lösevermitteln wie C1- bis C8-Alkoholen, Glycerin, Propylenglykol oder des wasserlöslichen Treibgases Dimethylether verschlechtern die Schaumeigenschaften drastisch.

Es stellte sich somit die Aufgabe, ein Mittel zur Erhöhung der Festigung und des Glanzes der Haare zu finden, welches diese Nachteile nicht aufweist.

Gelöst wird die Aufgabe durch die Verwendung eines speziellen, hochethoxylierten nichtionischen Tensids, welches bereits in geringen Mengen in einer Aerosolzusammensetzung zu einem Mittel führt, das sich ausgezeichnet ins Haar einarbeiten läßt und der Frisur eine gute Formbarkeit und einen langanhaltenden Glanz verleiht sowie dem Haar einen langanhaltenden weichen Griff verleiht.

Gegenstand der Erfindung ist daher die Verwendung eines bei Entnahme schaumbildenden Mittels mit einem Gehalt an
(A) mindestens einem Fettsäureglyceridpolyalkylenglykolether oder einem Fettsäurepartialglyceridpolyalkylenglykolether mit 70 bis 250 Alkylenglykoleinheiten und
(B) mindestens einem in Wasser löslichen oder unlöslichen Treibmittel, ausgewählt aus Dimethylether und gasförmigen Kohlenwasserstoffen oder deren Gemische zur Erhöhung der Formbarkeit und des Glanzes von Haaren.

Bei den Partialglyceriden kann es sich um Mono- oder Diglyceride oder um Mischungen aus Mono- und Diglyceriden handeln.

Komponente (A) ist ausgewählt aus Verbindungen der allgemeinen Formel (I)

R¹-(OA)_{X}-O-CH₂-CH[O-(AO)_{Y}-R²]-CH₂-O-(AO)_{Z}-R³ (I)

wobei R¹, R² und R³ unabhängig voneinander ausgewählt sind aus H und gesättigten oder ungesättigten, verzweigten oder unverzweigten C₆- bis C₂₂-Acylgruppen, wobei mindestens einer der Substituenten R¹, R² und R³ eine C₆- bis C₂₂-Acylgruppe ist; A eine Alkylengruppe mit zwei oder drei Kohlenstoffatomen ist; x,y und z Zahlen zwischen 0 und 250 sind, wobei die Summe x+y+z von 70 bis 250 beträgt. Besonders bevorzugt sind Verbindungen, bei denen R¹ gleich H ist, R² ausgewählt ist aus H und C₆bis C₂₂-Acylgruppen, R³ eine C₆- bis C₂₂-Acylgruppe bedeutet, A eine Ethylengruppe ist und x und y gleich 0 sind.

Beispiele für geeignete Verbindungen der Komponente (A) sind Polyethylenglykol(80)-glycerylcocoat, Polyethylenglykol(80)-glyceryltallowat, Polyethylenglykol(120)-glycerylstearat, Polyethylenglykol(200)-glycerylstearat, Polyethylenglykol(200)-glyceryltallowat, hydriertes Polyethylenglykol(200)-glycerylpalmitat. Besonders bevorzugt ist hydriertes Polyethylenglykol(200)-glycerylpalmitat.

Das erfindungsgemäß verwendete Mittel enthält Komponente (A) vorzugsweise zu 0,1 bis 30 Gewichtsprozent, besonders bevorzugt zu 0,3 bis 20 beziehungsweise 0,5 bis 12 Gewichtsprozent.

Bevorzugte wasserunlösliche Treibmittel sind flüchtige Kohlenwasserstoffe wie beispielsweise Propan und Butan und deren Mischungen sowie fluorierte Treibmittel.

Bevorzugtes wasserlösliches Treibmittel ist Dimethylether.

Das erfindungsgemäß verwendete Mittel liegt vorzugsweise in einer wässrigen oder in einer wässrig-alkoholischen Lösung sowie in Form einer einphasigen Aerosolzusammensetzung vor. Die Viskosität der Lösung ist vorzugsweise kleiner als 100 mm²/s bei 20 °C. Als Alkohole können insbesondere die für kosmetische Zwecke üblicherweise verwendeten niederen Alkohole mit 1 bis 4 Kohlenstoffatomen wie zum Beispiel Ethanol oder Isopropanol enthalten sein. Desweiteren können Lösungsmittel mit einem Siedepunkt unter 600 °C eingesetzt werden. Besonders geeignet sind dabei Propylenglykole sowie Glycerin. Die Lösungsmittel liegen in einer Menge von 0,01 bis 50 Gewichtsprozent, bevorzugt in einer Menge von 2 bis 30 Gewichtsprozent vor.

Das erfindungsgemäß verwendete Mittel kann zusätzlich wasserunlösliche Lösungsmittel enthalten, beispielsweise unverzweigte oder verzweigte Kohlenwasserstoffe wie Pentan, Hexan, Isopentan und zyklische Kohlenwasserstoffe wie Cyclopentan und Cyclohexan. Besonders bevorzugt sind Paraffine sowie Isododecan.

Als zusätzlichen Bestandteil kann das erfindungsgemäße Haarbehandlungsmittel als Komponente (C) mindestens ein weiteres nicht-ionisches Tensid in einer Menge von vorzugsweise 0,1 bis 30 Gewichtsprozent, besonders bevorzugt von 0,5 bis 20 Gewichtsprozent enthalten.

Bevorzugt sind dabei ethoxylierte Fettsäuren mit 10 bis 26 Kohlenstoffatomen, ethoxylierte Alkohole, ethoxylierte Fettalkohole mit 10 bis 26 Kohlenstoffatomen, ethoxyliertes hydriertes oder nicht hydriertes Rizinusöl, Glyceridalkoxylate, Fettsäureglyceridpolyalkylenglykolether oder Fettsäurepartialglyceridpolyalkylenglykolether mit jeweils weniger als 30 Alkylenglykoleinheiten wie beispielsweise Polyethylenglykol(7)-glycerylcocoat, Polyglykolamide, Fettsäurezuckerester, ethoxylierte Fettsäurezuckerester und Alkylpolyglycoside. Der Ethoxylierungsgrad von ethoxylierten Tensiden ist dabei vorzugsweise größer als 3.

Geeignete ethoxylierte Fettsäuren sind beispielsweise Polyethylenglykol(75)-laurat, Polyethylenglykol(90)-stearat, Polyethylenglykol(120)-stearat, Polyethylenglykol(120)-propylenglykolstearat, Polyethylenglykol(150)-dilaurat oder Polyethylenglykol(175)-distearat.

Geeignete ethoxylierte Fettsäurezuckerester sind ethoxylierte Sorbitanfettsäureester oder Polyethylenglykol(120)-methylglucosedioleat.

Als weiteren zusätzlichen Bestandteil kann das erfindungsgemäß verwendete Haarbehandlungsmittel als Komponente (D) mindestens ein filmbildendes, haarfestigendes Polymer in einer Menge von vorzugsweise 0,01 bis 10 Gewichtsprozent, besonders bevorzugt von 0,1 bis 8 Gewichtsprozent enthalten. Das Polymer kann synthetischen oder natürlichen Ursprungs und nichtionischer, anionischer, kationischer oder amphoterer Natur sein. Die haarfestigenden Polymere können einzeln oder im Gemisch eingesetzt werden.

Unter filmbildenden, haarfestigenden Polymeren werden erfindungsgemäß solche Polymere verstanden, die bei Anwendung in 0,1 bis 5%iger wäßriger, alkoholischer oder wäßrig-alkoholischer Lösung in der Lage sind, auf dem Haar einen Polymerfilm abzuscheiden und auf diese Weise das Haar zu festigen.

Als geeignete synthetische, nichtionische, filmbildende, haarfestigende Polymere können in dem erfindungsgemäß verwendeten Haarbehandlungsmittel Homopolymere des Vinylpyrrolidons, Homopolymere des N-Vinylformamids, Copolymere des Vinylpyrrolidons und Dimethylaminoalkylmethacrylaten, wobei Alkyl Methyl Ethyl oder Propyl bedeuten kann, Copolymerisate aus Vinylpyrrolidon und Vinylacetat, Terpolymere aus Vinylpyrrolidon, Vinylacetat und Vinylpropionat, Polyacrylamide, Polyvinylalkohole, oder Polyethylenglykol/Polypropylenglykol Copolymere eingesetzt werden.

Unter den geeigneten synthetischen, filmbildenden anionischen Polymeren sind beispielsweise Crotonsäure/Vinylacetat Copolymere, Vinylpyrrolidon/Vinylacrylat Copolymere, Terpolymere aus Acrylsäure, Ethylacrylat und N-t-Butylacrylamid und Methylvinylether/Maleinsäureanhydrid Copolymere und deren Monoester geeignet.

Natürliche filmbildende Polymere oder daraus durch chemische Umwandlung hergestellte Derivate können in dem erfindungsgemäß verwendeten Haarbehandlungsmittel ebenfalls eingesetzt werden. Bewährt haben sich niedermolekulares oder hochmolekulares Chitosan mit einem Molekulargewicht von 20.000 bis drei Millionen g/mol, Polysaccharide oder Gemische aus Oligo-, Mono- und Disacchariden (C-Pur® 01924 von Cerestar), chinesisches Balsamharz (Kolophonium), Cellulosederivate wie Hydroxypropylcellulose mit einem Molekulargewicht von 30.000 bis 50.000 g/mol, oder Schellack in neutralisierter oder unneutralisierter Form.

Auch amphotere Polymere können in dem erfindungsgemäß verwendeten Haarbehandlungsmittel eingesetzt werden. Amphotere Polymere besitzen im Molekül entweder sowohl freie basische Gruppen wie zum Beispiel Aminogruppen und freie saure Gruppen wie zum Beispiel Carbonsäure- oder Sulfonsäuregruppen und sind zur Bildung von inneren Salzen befähigt oder sie enthalten sowohl kationische Gruppen wie zum Beispiele quaternäre Ammoniumgruppen und anionische Gruppen wie beispielsweise Carboxylatoder Sulfat- oder Sulfonatgruppen. Geeignet sind insbesondere Copolymere aus Octylacrylamid, t-Butylaminoethylmethacrylat sowie zwei oder mehr Monomeren aus der Gruppe bestehend aus Acrylsäure, Methacrylsäure und deren einfachen Estern.

Zu den kationischen Polymeren, die erfindungsgemäß eingesetzt werden können, gehören Polymere, die entweder im Polymergerüst oder in Seitenketten kationische Gruppen, wie beispielsweise quaternäre Stickstoffatome tragen. Quaternäre Stickstoffatome können vier unterschiedliche oder zum Teil gleiche Substituenten tragen oder Teil ein Ringsystems sein. Bevorzugte kationische Gruppen sind Ammonium- oder Imidazoliniumgruppen. Die kationischen Polymere sind vorzugsweise Copolymerisate mit nichtionischen, polymerisierbaren vinylmonomeren, insbesondere Vinylpyrrolidon, Vinylacetat, Acrylamid, Methacrylamid, Methylacrylat, Ethylacrylat, Methylmethacrylat oder Ethylmethacrylat. Die Polymerhauptkette kann sich aber auch von Glykosiden oder Proteinen ableiten.

Geeignete kationische Polymere sind beispielsweise Copolymere des Vinylpyrrolidons mit quaternierten Derivaten des Dialkylaminoethylacrylats oder -methacrylats, Copolymerisate des Vinylpyrrolidons mit Vinylimidazoliummethochlorids, Polymere von Dimethyldiallylammoniumsalzen und deren Copolymere mit Estern oder Amiden von Acryl- oder Methacrylsäure, Kondensationsharze aus Polyglykolen und Polyaminen, kationisch derivatisierte Silikonöle, kationisch derivatisierte Proteinhydrolysate und quaternisierte Cellulose- oder Guarderivate.

Beispiele für solche Polymere sind das mit Diethylsulfat quaternierte Vinylpyrrolidon/Dimethylaminoethylmethacrylat Copolymer, das Copolymerisat des Vinylpyrrolidons mit Vinylimidazoliummethochlorid, das Terpolymer aus Dimethyldiallylammoniumchlorid, Natriumacrylat und Acrylamid, das Terpolymer aus Vinylpyrrolidon, Dimethylaminoethylmethacrylat und Vinylcaprolactam, das quaternierte Ammoniumsalz, hergestellt aus Hydroxyethylcellulose und einem mit Trimethylammonium substituierten Epoxid, das Vinylpyrrolidon/Methacrylamidopropyltrimethylammoniumchlorid Copolymer und diquaternäre Polydimethylsiloxane.

Des weiteren können Polymere mit verdickender Wirkung eingesetzt werden, sofern sie mit der Zusammensetzung des erfindungsgemäß verwendeten Mittels verträglich sind. Hierfür sind beispielsweise Homopolymere der Acrylsäure mit einem Molekulargewicht von 2.000.000 bis 6.000.000 g/mol geeignet. Auch Copolymere aus Acrylsäure und Acrylamid (Natriumsalz) mit einem Molekulargewicht von 2.000.000 bis 6.000.000 g/mol und Sclerotium Gum sind geeignet. Auch geeignet sind Copolymere der Acrylsäure und der Methacrylsäure sowie Copolymere aus Acrylat und/oder Methacrylatmonomeren und Acrylat- oder Methacrylatestern ethoxylierter C1- C30-Alkohole wie beispielsweise Acrylat/Steareth-20 Methacrylat Copolymer oder Steareth-10 Allylether/Acrylat Copolymer.

Die oben aufgeführten verdickenden oder filmbildenden anionischen oder amphoteren Polymere können teilweise oder ganz mit organischen oder anorganischen Basen neutralisiert werden. Bevorzugte Basen sind insbesondere primäre und sekundäre Amine, wie beispielsweise Aminomethylpropanol. Kationische Polymere, welche basische Gruppen enthalten, können mit organischen oder anorganischen Säuren ganz oder teilweise neutralisiert werden.

Des weiteren kann das erfindungsgemäß verwendeten Mittel wasserlösliche oder wasserunlösliche Silikonverbindungen in einer Konzentration von 0,01 bis 50 Gewichtsprozent, bevorzugt in einer Konzentration von 0,1 bis 5 Gewichtsprozent enthalten. Besonders bevorzugt sind dabei flüchtige und nichtflüchtige Cyclomethicone und Dimethicone sowie Dimethicon-Copolyole. Beispiele sind: Polydimethylsiloxan (INCI: Dimethicon), α-Hydro-ω-hydroxypolyoxydimethylsilylen (INC:Dimethiconol), cyclisches Dimethylpolysiloxan (INCI:Cyclomethicon), Trimethyl-(octadecyloxy)silan (INCI:Stearoxytrimethylsilan), Dimethylsiloxan-Glykol-Copolymer (INCI: Dimethicon Copolyol), Dimethylsiloxan/Aminoalkylsiloxan Copolymer mit Hydroxyendgruppen (INCI:Amodimethicon), Monomethylpolysiloxan mit Laurylseitenketten und Polyoxyethylen- und/oder Polyoxypropylenendketten, (INCI:Laurylmethicon Copolyol), Dimethylsiloxan-glykolcopolymeracetat (INCI:Dimethiconcopolyol Acetat) Dimethylsiloxan-aminoalkylsiloxan-Copolymer mit Trimethylsilylendgruppen (INCI: Trimethylsilylamodimethicon). Bevorzugte Silikonpolymere sind: Dimethicone, welche beispielsweise von der Firma Wacker, München, unter der Handelsbezeichnung Siloxane F-221 oder von der Firma Dow Corning Europe, Brüssel, unter der Handelsbezeichnung Dow Corning Fluid 200/0,65 cs vertrieben werden; Cyclomethicone, die beispielsweise unter den Handelsbezeichnungen Dow Corning 244 Fluid von der Firma Dow Corning Europe oder Abil® K4 von der Firma Goldschmidt vertrieben werden; Dimethiconole, die beispielsweise unter den Handelsbezeichnungen Silicone Fluid F-212 von der Firma Wacker oder Unisil® SF-R von der Firma UPI vertrieben werden.

Die vorstehend in Klammern angegebenen Bezeichnungen entsprechen der INCI Nomenklatur (International Cosmetic Ingredients), wie sie zur Kennzeichnung kosmetischer Wirk- und Hilfsstoffe bestimmt sind.

Auch Mischungen von Silikonpolymeren sind geeignet, wie z.B. eine Mischung aus Dimethicon und Dimethiconol, die beispielsweise unter der Handelsbezeichnung Dow Corning 1403 Fluid von der Firma Dow Corning Europe vertrieben wird.

Weitere geeignete Silikonpolymere sind Dimethicon Copolyole, welche unter den Handelsnamen Surfactant 193 von der Firma Dow Corning Europe oder Silwet® L von der Union Carbide vertrieben werden; Amodimethicone, die beispielsweise unter den Handelsnamen Sandoperm® FE von Sandoz oder SM 2059 von General Electric/USA vertrieben werden; Laurylmethicon Copolyol, das unter der Handelsbezeichnung Dow Corning Q2-5200 von der Firma Dow Corning Europe vertrieben wird; Trimethylsilylamodimethicone, die unter den Handelsbezeichnungen Dow Corning Q2-8220 von der Firma Dow Corning Europe oder Silicone Fluid F-801 von der Firma Wacker vertrieben werden; Dimethicon Copolyol Acetate, die unter den Handelsbezeichnungen Silicone Fluid VP oder Belsil® DMC 6033 von der Firma Wacker vertrieben werden und Trimethyl-(octadecyloxy)silane (INCI:Stearoxytrimethylsilane), die beispielsweise unter der Handelsbezeichnung Dow Corning 580 WAX von der Firma Dow Corning Europe vertrieben werden.

Das erfindungsgemäß verwendete Mittel kann Konsistenzgeber, wie sie üblicherweise für Cremes eingesetzt werden, enthalten, beispielsweise Fettalkohole und Fettalkoholsulfate wie sie unter der Bezeichnung Lanette® vertrieben werden. Ebenfalls enthalten sein können bei Raumtemperatur flüssige, wachsartige oder feste Polyethylenglykole.

Selbstverständlich kann das erfindungsgemäß verwendete Mittel auch weitere übliche kosmetische Zusätze, wie nichtfestigende, nichtionische Polymere, nichtfestigende, anionische Polymere und nichtfestigende, natürliche Polymere sowie deren Kombination in einer Menge von vorzugsweise 0,01 bis 15 Gewichtsprozent; Parfümöle in einer Menge von vorzugsweise 0,01 bis 5 Gewichtsprozent; Trübungsmittel wie z.B. Ethylenglykoldistearat, Styrol/PVP Copolymere oder Polystyrole in einer Menge von vorzugsweise 0,01 bis 5 Gewichtsprozent; Netzmittel, Tenside oder Emulgatoren mit oder ohne Waschaktivität aus den Klassen der anionischen, kationischen, amphoteren oberflächenaktiven Substanzen wie Fettalkoholsulfate, Fettalkoholethersulfate, Fettsäurealkanolamide in einer Menge von vorzugsweise 0,1 bis 20 Gewichtsprozent; ferner Feuchthaltemittel, Farbstoffe, Lichtschutzmittel, Antioxidantien, Glanzgeber und Konservierungsstoffe in einer Menge von vorzugsweise 0,01 bis 10 Gewichtsprozent enthalten.

Das erfindungsgemäß verwendete Mittel zeichnet sich insbesondere dadurch aus, daß es über längere Zeit stabile, transparente bis leicht opake Emulsionen mit wasserunlöslichen Stoffen wie z.B. Kohlenwasserstoffen oder Silikonölen bildet und sich bei Entnahme ein feinporiger, leichtverteilbarer Schaum bildet, der bei der Einarbeitung in das Haar zusammenbricht. Das erfindungsgemäß verwendete Mittel bewirkt eine Verbesserung der Formbarkeit und einen langanhaltenden Glanz und weichen Griff des Haars.

Die nachfolgenden Beispiele sollen den Gegenstand der Erfindung näher erläutern:

### Beispiele

### Beispiel 1: Haarbehandlungsmittel zur erleichterten Formgebung

| | |
|---|---|
| 0,75 g | hydriertes Polyethylenglykol(200)-glycerylpalmitat |
| 0,25 g | Polyethylenglykol(7)-glycerylcocoat |
| 9,0 g | Hydriertes Rizinusöl, ethoxyliert mit 40 mol Ethylenoxid |
| 84,0 g | Wasser |
| 6,0 g | Dimethylether |
| 100,00 g | |

### Beispiel 2: Haarbehandlungsmittel zur erleichterten Formgebung

| | |
|---|---|
| 5,25 g | hydriertes Polyethylenglykol(200)-glyceryltallowat |
| 1,75 g | Polyethylenglykol(7)-glycerylcocoat |
| 87,0 g | Wasser |
| 6,0 g | Dimethylether |
| 100,00 g | |

### Beispiel 3: Haarbehandlungsmittel mit Pflegeeffekt

| | |
|---|---|
| 0,7 g | hydriertes Polyethylenglykol(200)-glycerylpalmitat |
| 9,0 g | Hydriertes Rizinusöl, ethoxyliert mit 35 mol Ethylenoxid |
| 3,0 g | Cetyltrimethylammoniumchlorid |
| 1,0 g | Acrylat/Steareth-20 Methacrylat Copolymer (Acrysol® 22), mit Aminomethylpropanol neutralisiert auf pH 7 |
| 84,3 g | Wasser |
| 2.0 g | Propan/Butan, 2,7 bar |
| 100,00 g | |

### Beispiel 4: Haarbehandlungsmittel mit Pflegeeffekt

| | |
|---|---|
| 5,25 g | hydriertes Polyethylenglykol(200)-glycerylpalmitat |
| 1,75 g | Polyethylenglykol(7)-glycerylcocoat |
| 0,9 g | Hydriertes Rizinusöl, ethoxyliert mit 35 mol Ethylenoxid |
| 0,3 g | Glucosedecylether |
| 1,0 g | Amodimethicone |
| 84,8 g | Wasser |
| 6,0 g | Dimethylether |
| 100,00 g | |

### Beispiel 5: Haarbehandlungsmittel mit verstärktem Glanz

| | |
|---|---|
| 7,0 g | Polyethylenglykol(200)-glycerylstearat |
| 9,0 g | Hydriertes Polyethylenglykol(35)-Rizinusöl |
| 1,0 g | Polyethylenglykol(4)-laurylether |
| 1,0 g | Silikonöl AK 500 |
| 74,0 g | Wasser |
| 2,0 g | Propan/Butan, 2,7 bar |
| 6,0 g | Dimethylether |
| 100,00 g | |

### Beispiel 6: Haarbehandlungsmittel mit verstärktem Glanz und Schutz für das Haar

| | |
|---|---|
| 5,25 g | hydriertes Polyethylenglykol(200)-glyceryltallowat |
| 1,75 g | Polyethylenglykol(7)-glycerylcocoat |
| 9,0 g | Hydriertes Polyethylenglykol(35)-Rizinusöl |
| 1,0 g | Polyethylenglykol(4)-laurylether |
| 1,0 g | Dimethiconol, 13 %ig in Cyclomethicon (Dow Corning 1401 Fluid) |
| 0,3 g | Acrylat/C10-30-Alkylacrylat Crosspolymer (Pemulen® TR-1, Goodrich) |
| 73,7 g | Wasser |
| 2,0 g | Propan/Butan, 2,7 bar |
| 6,0 g | Dimethylether |
| 100,00 g | |

### Beispiel 8: Haarbehandlungsmittel mit schnelltrocknendem Effekt

| | |
|---|---|
| 7,0 g | Polyethylenglykol(80)-glycerylcocoat |
| 20,0 g | Ethanol |
| 65,0 g | Wasser |
| 2,0 g | Propan/Butan, 2,7 bar |
| 6,0 g | Dimethylether |
| 100,00 g | |

### Beispiel 9: Haarbehandlungsmittel mit schnelltrocknendem Effekt

| | |
|---|---|
| 7,0 g | Polyethylenglykol(80)-glyceryltallowat |
| 9,0 g | Hydriertes Rizinusöl, ethoxyliert mit 40 mol Ethylenoxid |
| 20,0 g | Ethanol |
| 56,0 g | Wasser |
| 2,0 g | Propan/Butan, 2,7 bar |
| 6,0 g | Dimethylether |
| 100,00 g | |

### Beispiel 10: Haarbehandlungsmittel mit UV-Schutz

| | |
|---|---|
| 0,5 g | Polyethylenglykol(200)-glycerylstearat |
| 9,0 g | Hydriertes Rizinusöl, ethoxyliert mit 40 mol Ethylenoxid |
| 0,5 g | Polyoxyethylen(25)-p-aminobenzoesäure |
| 82,0 g | Wasser |
| 2,0 g | Propan/Butan, 2,7 bar |
| 6,0 g | Dimethylether |
| 100,00 g | |

### Beispiel 11: Haarbehandlungsmittel mit Festigung

| | |
|---|---|
| 7,0 g | hydriertes Polyethylenglykol(200)-glycerylpalmitat |
| 0,2 g | Polyoxyethylen(120)-Methylglucosedioleat |
| 4,0 g | Polyvinylpyrrolidon (Luvsikol® K 60, BASF) |
| 80,8 g | Wasser |
| 2,0 g | Propan/Butan, 2,7 bar |
| 6,0 g | Dimethylether |
| 100,00 g | |

### Beispiel 12: Haarbehandlungsmittel mit Festigung

| | |
|---|---|
| 5,25 g | hydriertes Polyethylenglykol(200)-glycerylpalmitat |
| 1,75 g | Polyethylenglykol(7)-glycerylcocoat |
| 0,3 g | Polyethylenglykol(23)-laurylether |
| 2,0 g | Vinylacetat/Crotonat Copolymer (Luviset® CA-66, BASF) |
| 82,7 g | Wasser |
| 2,0 g | Propan/Butan, 2,7 bar |
| 6,0 g | Dimethylether |
| 100,00 g | |

### Beispiel 13: Haarbehandlungsmittel mit Festigung

| | |
|---|---|
| 7,0 g | Polyethylenglykol(80)-glyceryltallowat |
| 4,0 g | Vinylpyrrolidon/Dimethylaminoethylmethacrylat Methosulfat Copolymer, 20%ig in Wasser (Gafquat® 755 N, ISP) |
| 81,0 g | Wasser |
| 2,0 g | Propan/Butan, 2,7 bar |
| 6,0 g | Dimethylether |
| 100,00 g | |

### Beispiel 14: Haarbehandlungsmittel mit Festigung

| | |
|---|---|
| 5,25 g | hydriertes Polyethylenglykol(200)-glyceryltallowat |
| 1,75 g | Polyethylenglykol(7)-glycerylcocoat |
| 9,0 g | Hydriertes Rizinusöl, ethoxyliert mit 40 mol Ethylenoxid |
| 1,0 g | Octylacrylamid/Acrylat/Butylaminoethylmethacrylat Copolymer (Amphomer®, National Starch) |
| 75,0 g | Wasser |
| 2,0 g | Propan/Butan, 2,7 bar |
| 6,0 g | Dimethylether |
| 100,00 g | |

### Beispiel 15: Haarbehandlungsmittel mit Festigung

| | |
|---|---|
| 5,25 g | hydriertes Polyethylenglykol(200)-glyceryltallowat |
| 1,75 g | Polyethylenglykol(7)-glycerylcocoat |
| 9,0 g | Hydriertes Rizinusöl, ethoxyliert mit 40 mol Ethylenoxid |
| 0,6 g | Chitosan |
| 75,4 g | Wasser |
| 2,0 g | Propan/Butan, 2,7 bar |
| 6,0 g | Dimethylether |
| 100,00 g | |

### Beispiel 16: Haarbehandlungsmittel mit Pflegepolymer

| | |
|---|---|
| 5,25 g | hydriertes Polyethylenglykol(200)-glycerylpalmitat |
| 1,75 g | Polyethylenglykol(7)-glycerylcocoat |
| 0,3 g | Hydroxypropyl-guar-hydroxypropyltrimethylammoniumchlorid (Jaguar® C 162, Rhöne-Poulenc) |
| 84,7 g | Wasser |
| 2,0 g | Propan/Butan, 2,7 bar |
| 6,0 g | Dimethylether |
| 100,00 g | |

## Patentansprüche

1. Verwendung eines bei Entnahme schaumbildenden Mittels mit einem Gehalt an
(A) mindestens einem Fettsäureglyceridpolyalkylenglykolether oder einem Fettsäurepartialglyceridpolyalkylenglykolether mit 70 bis 250 Alkylenglykoleinheiten und
(B) mindestens einem in Wasser löslichen oder unlöslichen Treibmittel, ausgewählt aus Dimethylether und gasförmigen Kohlenwasserstoffen oder deren Gemische
zur Erhöhung der Formbarkeit und des Glanzes von Haaren, wobei Komponente (A) ausgewählt ist aus Verbindungen der allgemeinen Formel (I)
R¹-(OA)ₓ-O-CH₂-CH[O-(AO)_{Y}-R²]-CH₂-O-(AO)_{Z}-R³ (I)
wobei R¹, R² und R³ unabhängig voneinander ausgewählt sind aus H und gesättigten oder ungesättigten, verzweigten oder unverzweigten C₆- bis C₂₂-Acylgruppen, wobei mindestens einer der Substituenten R¹, R² und R³ eine C₆- bis C₂₂-Acylgruppe ist; A eine Alkylengruppe mit zwei oder drei Kohlenstoffatomen ist; x,y und z Zahlen zwischen 0 und 250 sind, wobei die Summe x+y+z von 70 bis 250 beträgt:

2. Verwendung nach Anspruch 1, **dadurch gekennzeichnet, dass** R¹ gleich H ist, R² ausgewählt ist aus H und C₆-bis C₂₂-Acylgruppen, R³ eine C₆- bis C₂₂-Acylgruppe bedeutet, A eine Ethylengruppe ist und x und y gleich 0 sind.

3. Verwendung nach einem der vorstehenden Ansprüche, **dadurch gekennzeichnet, dass** Komponente (A) hydriertes Glycerylpalmitat mit rund 200 Polyethylenglykoleinheiten ist.

4. Verwendung nach einem der vorstehenden Ansprüche, **dadurch gekennzeichnet, dass** das Mittel 0,1 bis 30 Gewichtsprozent der Komponente (A) enthält.

5. Verwendung nach einem der vorstehenden Ansprüche, **dadurch gekennzeichnet, dass** das Mittel als Komponente (C) mindestens ein zusätzliches, nichtionisches Tensid enthält.

6. Verwendung nach Anspruch 5, **dadurch gekennzeichnet, dass** Komponente (C) ausgewählt ist aus ethoxylierten Fettsäuren, ethoxylierten Alkoholen, ethoxylierten Fettalkoholen, Glyceridalkoxylaten, Fettsäureglyceridpolyalkylenglykolethern oder Fettsäurepartialglyceridpolyalkylenglykolethern mit jeweils weniger als 30 Alkylenglykoleinheiten, Polyglykolamiden, Fettsäurezuckerestern, ethoxylierten Fettsäurezuckerestern und Alkylpolyglycosiden.

7. Verwendung nach einem der vorstehenden Ansprüche, **dadurch gekennzeichnet, dass** das Mittel 0,1 bis 30 Gewichtsprozent der Komponente (C) enthält.

8. Verwendung nach einem der vorstehenden Ansprüche, **dadurch gekennzeichnet, dass** das Mittel als wässrige oder wässrig-alkoholische Lösung vorliegt.

9. Verwendung nach einem der vorstehenden Ansprüche, **dadurch gekennzeichnet, dass** das Mittel eine Viskosität von kleiner 100 mm²/s bei 20 °C aufweist.

10. Verwendung nach einem der vorstehenden Ansprüche, **dadurch gekennzeichnet, dass** das Mittel als einphasige Aerosolzusammensetzung vorliegt.

11. Verwendung nach einem der vorstehenden Ansprüche, **dadurch gekennzeichnet, dass** das Mittel als Komponente (D) zusätzlich ein filmbildendes, haarfestigendes Polymer enthält.

12. Verwendung nach Anspruch 11, **dadurch gekennzeichnet, dass** das Mittel 0,01 bis 10 Gewichtsprozent der Komponente (D) enthält.

## Claims

1. Use of an agent that foams upon extraction, with a content of
(A) at least one fatty acid glyceride polyalkylene glycol ether or a fatty acid partial glyceride polyalkylene glycol ether with 7 to 250 alkylene glycol units, and
(B) at least one foaming agent that is soluble or insoluble in water, selected from dimethyl ether and gaseous hydrocarbons or their mixtures
to increase the shaping capability and the glossiness of hair, wherein component (A) is selected from compounds of the general formula (I)
R¹-(OA)ₓ-O-CH₂-CH[O-(AO)_{Y}-R²]-CH₂-O-(AO)_{Z}-R³ (I)
wherein R¹, R² and R³ are selected independently of one another from H and saturated or unsaturated, branched or unbranched C₆- to C₂₂-acyl groups, wherein at least one of the substituents R¹, R² and R³ is a C₆- to C₂₂-acyl group; A is an alkylene group with two or three carbon atoms; x, y and z are numbers between 0 and 250, wherein the sum x+y+z is from 70 to 250.

2. Use according to claim 1, **characterized in that** R¹ is equal to H; R2 is selected from H and C₆- to C₂₂-acyl groups; R³ means a C₆- to C₂₂-acyl group; A is an ethylene group; and x and y are equal to 0.

3. Use according to any one of the preceding claims, **characterized in that** component (A) is hydrogenated glyceryl palmitate with approximately 200 polyethylene glycol units.

4. Use according to any one of the preceding claims, **characterized in that** the agent includes 0.1 to 30 percent by weight of component (A).

5. Use according to any one of the preceding claims, **characterized in that** the agent includes at least one additional, nonionic surfactant as component (C).

6. Use according to claim 5, **characterized in that** component (C) is selected from ethoxylated fatty acids, ethoxylated alcohols, ethoxylated fatty alcohols, glyceride alkoxylates, fatty acid glyceride polyalkylene glycol ethers or fatty acid partial glyceride polyalkylene glycol ethers with respectively fewer than 30 alkylene glycol units, polyglycol amides, fatty acid sucrose esters, ethoxylated fatty acid sucrose esters, and alkyl polyglycosides.

7. Use according to any one of the preceding claims, **characterized in that** the agent includes 0.1 to 30 percent by weight of component (C).

8. Use according to any one of the preceding claims, **characterized in that** the agent exists as an aqueous or aqueous alcoholic solution.

9. Use according to any one of the preceding claims, **characterized in that** the agent has a viscosity of less than 100 mm²/s at 20 °C.

10. Use according to any one of the preceding claims, **characterized in that** the agent exists as a single-phase aerosol composition.

11. Use according to any one of the preceding claims, **characterized in that** the agent additionally includes a film-forming, hair-setting polymer as component (D).

12. Use according to claim 11, **characterized in that** the agent includes 0.1 to 10 percent by weight of component (D).

## Revendications

1. Utilisation d'un agent moussant lors du prélèvement, présentant une teneur en
(A) au moins un glycéride-polyalkylèneglycoléther d'acide gras ou un glycéride-polyalkylèneglycoléther partiel d'acide gras comportant de 7 à 250 groupes alkylèneglycol et
(B) au moins un agent propulseur soluble ou insoluble dans l'eau, choisi parmi l'éther diméthylique et des hydrocarbures gazeux ou des mélanges de ceux-ci
permettant l'amélioration de la mise en forme et de la brillance des cheveux, le composant (A) étant choisi parmi des composés de la formule générale (I)
R¹-(OA)ₓ-O-CH₂-CH[O-(AO)_{Y}-R²]-CH₂-O-(AO)_{Z}-R³ (I)
dans laquelle R¹, R² et R³ sont choisis, indépendamment les uns des autres, parmi H et des groupes acyle en C₆-C₂₂ saturés ou insaturés, ramifiés ou non ramifiés, au moins un des substituants R¹, R² et R³ étant un groupe acyle en C₆-C₂₂ ; A est un groupe alkylène ayant deux ou trois atomes de carbone ; x, y et z sont des nombres compris entre 0 et 250, la somme x+y+z allant de 70 à 250.

2. Utilisation selon la revendication 1, **caractérisée en ce que** R¹ est H, R² est choisi parmi H et des groupes acyle en C₆-C₂₂, R³ représente un groupe acyle en C₆-C₂₂, A est un groupe éthylène et x et y valent 0.

3. Utilisation selon l'une des revendications précédentes, **caractérisée en ce que** le composant (A) est un palmitate de glycéryle hydrogéné comportant environ 200 groupes polyéthylèneglycol.

4. Utilisation selon l'une des revendications précédentes, **caractérisée en ce que** l'agent contient de 0,1 à 30 % en poids du composant (A).

5. Utilisation selon l'une des revendications précédentes, **caractérisée en ce que** l'agent contient en tant que composant (C) au moins un tensioactif non ionique supplémentaire.

6. Utilisation selon la revendication 5, **caractérisée en ce que** le composant (C) est choisi parmi des acides gras éthoxylés, des alcools éthoxylés, des alcools gras éthoxylés, des alcoxylates de glycérides, des glycéride-polyalkylèneglycoléthers d'acides gras ou glycéride-polyalkylèneglycoléthers partiels d'acides gras comportant chacun moins de 30 groupes alkylèneglycol, des polyglycolamides, des esters glucidiques d'acides gras, des esters glucidiques d'acides gras éthoxylés et des alkylpolyglycosides.

7. Utilisation selon l'une des revendications précédentes, **caractérisée en ce que** l'agent contient de 0,1 à 30 % en poids du composant (C).

8. Utilisation selon l'une des revendications précédentes, **caractérisée en ce que** l'agent se trouve sous forme de solution aqueuse ou aqueuse-alcoolique.

9. Utilisation selon l'une des revendications précédentes, **caractérisée en ce que** l'agent présente une viscosité inférieure à 100 mm²/s à 20 °C.

10. Utilisation selon l'une des revendications précédentes, **caractérisée en ce que** l'agent se trouve sous forme d'une composition monophasique pour aérosol.

11. Utilisation selon l'une des revendications précédentes, **caractérisée en ce que** l'agent contient également, en tant que composant (D), un polymère filmogène fixant le cheveu.

12. Utilisation selon la revendication 11, **caractérisée en ce que** l'agent contient de 0,01 à 10 % en poids du composant (D).
